# EUROPÄISCHE PATENTSCHRIFT

(11) **EP 4 523 527 B1**
(45) Veröffentlichungstag und Bekanntmachung des Hinweises auf die Patenterteilung: **25.03.2026**
(21) Anmeldenummer: 23197147.4
(22) Anmeldetag: 13.09.2023
(51) Int. Cl.: A01K 67/36

(54) **FLIEGENKÄFIGMODUL MIT FÖRDERBAND**
FLY CAGE MODULE WITH CONVEYOR BELT
MODULE DE CAGE DE MOUCHES AVEC BANDE TRANSPORTEUSE

(43) Veröffentlichungstag der Anmeldung: 19.03.2025
(73) Patentinhaber: Livin Farms Agrifood GmbH, 1110 Wien (AT)
(72) Erfinder: Unger, Katharina, 1120 Wien (AT); Wixler, Jürgen, 49201 Dissen am Teutoburger Wald (DE)
(74) Vertreter: Patentanwälte Pinter & Weiss OG

(56) Entgegenhaltungen:
- WO-A1-2021/130517
- WO-A1-2023/089142
- CN-A- 105 340 843
- CN-A- 112 335 611
- FR-A1- 3 066 360
- FR-A1- 3 081 677
- US-A1- 2019 191 678
- US-A1- 2022 304 289
- US-A1- 2022 408 705

## Beschreibung

Die gegenständliche Erfindung betrifft eine Vorrichtung zur Produktion von Insekteneiern, wobei ein Behälter vorgesehen ist, welcher dazu ausgebildet ist, Insekten, insbesondere als erwachsene Insekten, in einem Behälterinnenraum des Behälters aufzunehmen und die aufgenommenen Insekten daran hindert, den Behälter zu verlassen, wobei im Behälter zumindest eine Eiablagevorrichtung vorgesehen ist, welche dazu ausgebildet ist, Insekteneier, welche bei Verwendung des Behälters durch die Insekten im Behälter abgelegt werden, aufzunehmen, wobei die zumindest eine Eiablagevorrichtung zumindest teilweise aus dem Behälter entfernbar ist. Die gegenständliche Erfindung betrifft weiters eine Anordnung und ein Verfahren zur Produktion von Insekteneiern.

Ein Lebenszyklus eines Insekts beschreibt verschiedene Wachstumsstadien (beispielsweise Ei, Larve, Puppe und erwachsenes Insekt), wobei die Dauer dieser Wachstumsstadien und die Wachstumsstadien je nach Art des Insekts variieren. Der Begriff "Insekt" umfasst dabei das Insekt in den verschiedenen Wachstumsstadien. Je nach Art der Insekten können die Wachstumsphasen einige Tage bis einige Wochen in Anspruch nehmen. In der industriellen Insektenzucht werden die Insekten insbesondere für Lebensmittel und für Tierfutter gezüchtet. Von der Produktion der Insekteneier bis hin zum Heranzüchten der erwachsenen Insekten, deckt die industrielle Insektenzucht die verschiedenen Wachstumsstadien der Insekten ab. Nachdem die Insekten als Larven aus den Eiern schlüpfen, werden diese üblicherweise in mit Substrat gefüllten Behältern oder Boxen in vorgegebene Umgebungsbedingungen gelagert, wo diese zu erwachsenen Insekten heranwachsen. Während der Lagerung in den Behältern können sich die Larven auch zur Puppe verpuppen, woraus die erwachsenen Insekten heranwachsen. Das Substrat umfasst dabei Futter für die Larven. Die erwachsenen Insekten werden "geerntet", also aus den Behältern entfernt, und können anschließend, z.B. zu proteinreichen Endprodukten wie Öl oder Pulver, weiterverarbeitet werden. Vergleichbar mit anderen Branchen werden Produktionsanlage in der industriellen Insektenzucht ebenfalls großteils automatisiert betrieben.

In FR 3 066 360 A1, WO 2023/089142 A1, US 2022/408705 A1 und CN 112 335 611 A werden Vorrichtungen zur Produktion von Insekteneiern unter Verwendung von Lockmitteln offenbart.

In IN 2020/11046203 A wird ein System offenbart, mit welchem die Produktion der Insekteneier automatisiert abläuft. Dabei sind zwei Arten von Kammern vorgesehen, wobei in einer ersten Kammer Insekten (schwarze Soldatenfliegen) gezüchtet werden. In einer zweiten Kammer sind Eiablagevorrichtung vorgesehen, in welche die gezüchteten schwarzen Soldatenfliegen die Eier legen. Diese Eiablagevorrichtungen sind wabenförmig aufgebaut und befinden sich an den Wänden der zweiten Kammer. Dabei werden die Umgebungsbedingungen, wie etwa die Temperatur, das Licht oder die Luftfeuchtigkeit, in den Kammern speziell für die Haltung, für die Fortpflanzung und für die Eiablage der Insekten geregelt.

In WO 2022/081014 A1 ist ebenfalls ein System zur Produktion von Insekteneiern offenbart, wobei ein Förderband mit einem Substrat als Eiablagevorrichtung verwendet wird. Über dem Förderband sind bewegbare Behälter angeordnet, in welchen Insekten als erwachsene Insekten gehalten werden. An der Unterseite der Behälter ist ein feines Gitter vorhanden. Dadurch werden einerseits die in den Behältern gehaltenen erwachsenen Insekten daran gehindert, die Behälter zu verlassen. Andererseits wird auf dem Förderband das Substrat für die Eiablage gefördert und durch Herablassen der Behälter auf das Förderband, taucht das feine Gitter in das Substrat ein, wodurch das Substrat in die Behälter gelangen kann. Die erwachsenen Insekten legen anschließend die Eier in das Substrat auf dem Förderband ab. Nach der Eiablage werden die Behälter vom Förderband wieder angehoben und die Eier können vom Förderband geerntet werden.

Bei der Eiablage kann es dazu kommen, dass die erwachsenen Insekten die Eier nicht in die dafür vorgesehenen Eiablagevorrichtungen ablegen, sondern in andere Öffnungen, Kanten, Ecken, usw., welche in den Kammern oder in den Behältern vorhanden sind. Dadurch erhöht sich der Aufwand, um diese Eier zu ernten bzw. sind die Eiablagevorrichtungen dementsprechend nur zu einem Bruchteil mit Eiern gefüllt.

Es ist daher eine Aufgabe der gegenständlichen Erfindung die Produktion von Insekteneiern zu optimieren, insbesondere die Eiablage zu verbessern und die Ernte der Eier zu vereinfachen.

Die Erfindung ist im beigefügten Anspruchssatz beschrieben.

Erfindungsgemäß wird die Aufgabe mit einer Vorrichtung zur Produktion von Insekteneiern dadurch gelöst, dass im Bereich der zumindest einen Eiablagevorrichtung zumindest ein Lockmittel angeordnet ist, welches Reizstoffe für die Insekten abgibt, um bei Verwendung des Behälters die Insekten zum Ablegen der Insekteneier zu der zumindest einen Eiablagevorrichtung zu locken, wobei das zumindest eine Lockmittel von der zumindest einen Eiablagevorrichtung örtlich getrennt ist und vom Behälterinnenraum abgetrennt ist, wobei die vom Lockmittel abgegebenen Reizstoffe durch die zumindest eine Eiablagevorrichtung in den Behälterinnenraum gelangen. Dadurch werden bei Verwendung der Vorrichtung die Insekten im Behälter angeregt, die Insekteneier in die zumindest eine Eiablagevorrichtung abzulegen. Folglich erhöht sich einerseits die Ausbeute an Insekteneiern und andererseits verringert sich der Aufwand, um die Insekteneier zu ernten. Vorzugsweise ist das zumindest eine Lockmittel Wasser und/oder tote Insekten. Somit können auch die toten Insekten weiterverwendet werden, welche sich z.B. am Boden des Behälters ansammeln.

In einer bevorzugten Ausführungsform ist die zumindest eine Eiablagevorrichtung perforiert. Dadurch dass die Insekten es vorziehen die Insekteneier insbesondere in Öffnungen abzulegen, ist eine Perforierung der zumindest einen Eiablagevorrichtung für die Eiablage vorteilhaft.

Erfindungsgemäß ist die zumindest eine Eiablagevorrichtung auf einem Förderband angeordnet. Dadurch verringert sich der Arbeitsaufwand, um beispielsweise die zumindest eine Eiablagevorrichtung für die Ernte der Insekteneiern aus dem Behälter zu befördern.

Vorteilhafterweise ist die zumindest eine Eiablagevorrichtung zumindest teilweise vom Förderband entfernbar. Somit ist es möglich, beispielsweise zur Ernte der Insekteneier, nur Teile der zumindest einen Eiablagevorrichtung vom Förderband entfernt werden, wodurch sich die Handhabung der zumindest einen Eiablagevorrichtung verbessert.

In einer bevorzugten Ausführungsform weist der Behälter zumindest eine Auslassöffnung auf, wobei sich die zumindest eine Eiablagevorrichtung auf dem Förderband zumindest teilweise durch die zumindest eine Auslassöffnung hindurch erstreckt. Durch die zumindest eine Auslassöffnung kann auf ein Betreten des Behälters zur Ernte der Insekteneier aus der zumindest einen Eiablagevorrichtung verzichtet werden.

Vorzugsweise ist als das zumindest eine Lockmittel Wasser unterhalb des Förderbandes angeordnet. Dadurch dass die Insekten es vorziehen in der Nähe von Wasser die Insekteneier abzulegen, ist die Position des Wassers unterhalb des Förderbandes vorteilhaft.

In einer vorteilhaften Ausführungsform umfasst der Behälter zumindest eine Insektenfördereinrichtung, welche dazu ausgebildet ist, die Insekten in den Behälter zu fördern. Dadurch kann auf ein manuelles Einbringen der Insekten in den Behälter verzichtet werden, wodurch sich der Aufwand reduziert. Zudem kann die Insektenfördereinrichtung ein kontinuierliches und selbstständiges Einbringen der Insekten in den Behälter ermöglichen.

In einer weiteren vorteilhaften Ausführungsform weist der Behälter eine Sammeleinrichtung auf, welche dazu ausgebildet ist, die toten Insekten in dem Behälter zu sammeln und im Bereich der zumindest einen Eiablagevorrichtung als das zumindest eine Lockmittel anzuordnen. Somit wird einerseits eine selbstständige Reinigung der Behälterinnenraum ermöglicht, wodurch sich die Hygiene erhöht. Andererseits können die toten Insekten als das zumindest eine Lockmittel weiterverwendet werden.

In einer bevorzugten Anordnung zur Produktion von Insekteneiern ist eine Mehrzahl an Vorrichtungen aneinandergereiht und/oder gestapelt angeordnet. Für ein erhöhtes Produktionsvolumen der Insekteneier können die Vorrichtungen, je nach Kundenwunsch, einfach und platzsparend angeordnet bzw. erweitert werden.

Bei einem erfindungsgemäßen Verfahren mit der Vorrichtung zur Produktion von Insekteneiern wird zumindest ein Lockmittel im Bereich der zumindest einen Eiablagevorrichtung angeordnet und die Insekten durch das Lockmittel zum Ablegen der Insekteneier zu der zumindest eine Eiablagevorrichtung gelockt werden. Die Insekten im Behälter werden dadurch zu einer gezielteren Eiablage in die zumindest eine Eiablagevorrichtung gelenkt, wodurch sich die Ausbeute der Insekteneier erhöht. Dadurch vereinfacht sich zusätzlich die Ernte der Insekteneier.

Vorteilhafterweise werden tote Insekten im Behälter mit einer Sammeleinrichtung gesammelt und die toten Insekten werden als das zumindest eine Lockmittel der zumindest einen Eiablagevorrichtung bereitgestellt. Dadurch werden einerseits die toten Insekten entfernt, um den Behälterinnenraum sauber und hygienisch zu halten. Andererseits können die gesammelten toten Insekten einfach als das zumindest eine Lockmittel weiterverwendet werden.

Bevorzugt werden in vorgegebenen Zeitabständen, vorzugsweise einmal pro Tag, die Insekteneier aus der zumindest einen Eiablagevorrichtung entfernt. Damit kann eine kontinuierliche Produktion der Insekteneier erzielt werden.

Vorteilhafterweise werden die Insekteneier mechanisch oder pneumatisch oder durch Ausspülen mit einer Flüssigkeit aus der zumindest einen Eiablagevorrichtung entfernt. Dadurch können die Insekteneier einfach entfernt werden, insbesondere wenn diese an der Eiablagevorrichtung haften.

Die Insekten werden vorzugsweise mit zumindest einer Insektenfördereinrichtung in den Behälter gefördert. Dadurch kann der Aufwand reduziert werden, da auf ein manuelles Einbringen der Insekten in den Behälter verzichtet wird. Zusätzlich kann ein kontinuierliches und selbstständiges Einbringen der Insekten in den Behälter realisiert werden ohne den Behälter betreten zu müssen.

Die gegenständliche Erfindung wird nachfolgend unter Bezugnahme auf die Figuren 1 bis 4 näher erläutert, die beispielhaft, schematisch und nicht einschränkend vorteilhafte Ausgestaltungen der Erfindung zeigen. Dabei zeigt
Fig. 1 den grundlegenden Aufbau einer Vorrichtung,
Fig. 2 eine erfindungsgemäße Vorrichtung,
Fig. 3 ein beispielhaftes Verfahren zur Produktion von Insekteneiern, und
Fig. 4 eine bevorzugte Anordnung der erfindungsgemäßen Vorrichtung.

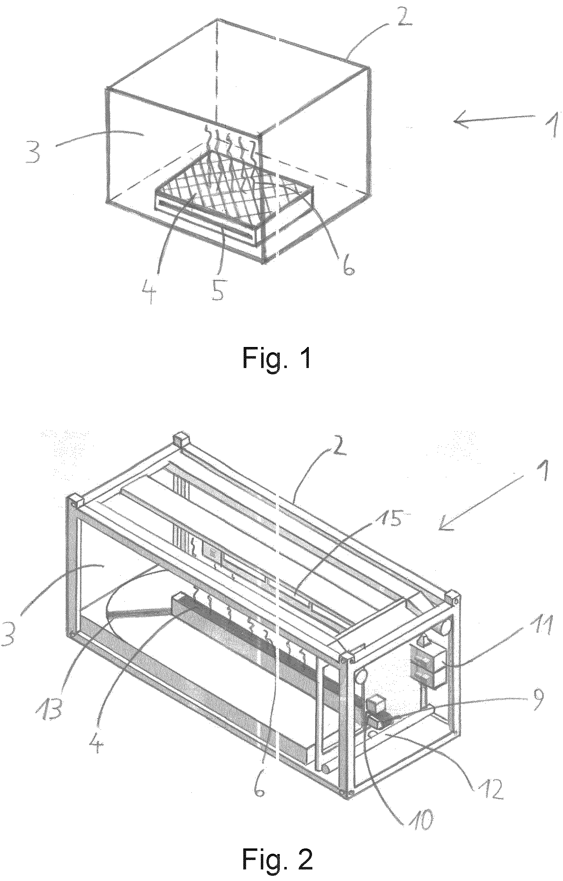

In Fig. 1 ist der grundlegende Aufbau einer Vorrichtung 1 zur Produktion von Insekteneiern 7 dargestellt. Dabei ist ein Behälter 2 vorgesehen, welcher dazu ausgebildet ist, Insekten 8, insbesondere als erwachsene Insekten 8 (z.B. als Fliegen), in einem Behälterinnenraum 3 des Behälters 2 aufzunehmen und die aufgenommenen Insekten 8 daran zu hindern, den Behälter 2 zu verlassen.

Der Behälter 2 kann beispielsweise wie in Fig. 1 dargestellt rechteckförmig sein (z.B. ein Container), wobei natürlich auch andere Formen des Behälters 2 möglich sind. Den Behälterinnenraum 3 in Fig. 1 bilden dabei grundsätzlich vier Behälterwände, eine Behälterdecke und ein Behälterboden, wobei diese unterschiedlich ausgeführt sein können. Beispielsweise können diese aus unterschiedlichen Materialen, wie etwa Kunststoff (z.B. Acrylglas), Metall, Holz, etc. ausgeführt sein, wobei sichergestellt werden soll, dass die Insekten 8 den Behälter 2 nicht verlassen können. Der Behälter 2 kann auch aus einem Rahmen (z.B. aus Metall) aufgebaut sein, wobei die Behälterwände und die Behälterdecke beispielsweise aus einem feinmaschigen Netz (z.B. Mosquitonetz) ausgeführt sind.

Je nach Material für die Behälterwände, die Behälterdecke und den Behälterboden kann eine Belüftung und/oder Beleuchtung des Behälterinnenraums 3 (nicht dargestellt) vorgesehen sein. Damit können bei Verwendung der Vorrichtung 1 für die Insekten 8 Bedingungen im Behälterinnenraum 3 geschaffen werden, welche das Überleben der Insekten 8, zumindest für einen gewissen Zeitraum, sicherstellen und die Eiablage fördern (z.B. eine optimale Luftzufuhr, Temperatur, Feuchtigkeit, Lichtstärke, etc., im Behälter 2).

Der Behälter 2 kann eine verschließbare Öffnung, wie eine Klappe, Tür, abnehmbare Wand usw., aufweisen, um über die Öffnung den Behälterinnenraum 3 bedarfsweise zugänglich zu machen, beispielsweise zur Reinigung, um Insekten 8 in den Behälter 2 zu geben oder um die Eiablagevorrichtung 4 zu entnehmen.

In Fig. 1 ist im Behälter 2 beispielhaft eine Eiablagevorrichtung 4 dargestellt, welche dazu ausgebildet ist, Insekteneier 7, welche bei Verwendung des Behälters 2 durch die Insekten 8 im Behälter 2 abgelegt werden, aufzunehmen. Natürlich kann auch eine Mehrzahl an Eiablagevorrichtungen 4 im Behälter 2 vorgesehen sein. Die Eiablagevorrichtung 4 kann, wie in Fig. 1 dargestellt, als Matte ausgeführt sein, wobei die Eiablagevorrichtung 4 vorzugsweise perforiert ist, z.B. kreisförmige Ausnehmungen (nicht dargestellt) aufweist. Die Eiablagevorrichtung 4 kann beispielsweise aus Kunststoff, wie etwa Polyethylen, Polypropylen oder Acrylnitril-Butadien-Styrol-Copolymere ausgeführt sein. Weiters ist die Eiablagevorrichtung 4 vorzugsweise zumindest teilweise aus dem Behälter 2 entfernbar. Die Eiablagevorrichtung 4 könnte auch unterteilt sein und aus einer Mehrzahl an Eiablagevorrichtungseinheiten (z.B. Segmente der Matte) bestehen, welche aus der Eiablagevorrichtung 4 und aus dem Behälter 2 entnommen werden können, um die Insekteneier zu ernten. Die Eiablagevorrichtung 4 kann auch gefärbt sein, um bei Verwendung des Behälters 2 die Insekten zur Eiablagevorrichtung 4 zu locken. Die Eiablagevorrichtung 4 kann zusätzlich auch beheizt sein, um bei Verwendung des Behälters 2 die Insekten zur Eiablagevorrichtung 4 zu locken.

Wie in Fig. 1 dargestellt, ist im Bereich der Eiablagevorrichtung 4 zumindest ein Lockmittel 5 angeordnet, welches Reizstoffe 6 für die Insekten 8 abgibt, um bei Verwendung des Behälters 2 die Insekten 8 zum Ablegen der Insekteneier 7 zu der Eiablagevorrichtung 4 zu locken. Das Lockmittel 5 ist von der zumindest einen Eiablagevorrichtung 4 örtlich getrennt und vom Behälterinnenraum 3 abgetrennt, wobei die vom Lockmittel 5 abgegebenen Reizstoffe 6 durch die zumindest eine Eiablagevorrichtung 4 in den Behälterinnenraum 4 gelangen. "Vom Behälterinnenraum 3 abgetrennt" bedeutet in diesem Zusammenhang, dass verhindert ist, dass Insekten 8 aus dem Behälterinnenraum 3 zum Lockmittel 5 gelangen können. "Örtlich getrennt" bedeutet in diesem Zusammenhang, dass die Eiablagevorrichtung 4 getrennt vom Lockmittel 5 entfernt werden kann. Wie in Fig. 1 nur beispielhaft angedeutet, ist das Lockmittel 5 in einer Box unterhalb der Eiablagevorrichtung 4 angeordnet, wobei die Eiablagevorrichtung 4 die Box nach oben begrenzt. Die durch das Lockmittel 5 abgegeben Reizstoffe 6 können, beispielsweise durch Öffnungen in der Eiablagevorrichtung 4, durch die Eiablagevorrichtung 4 in den Behälterinnenraum 3 gelangen. Durch die Reizstoffe 6 werden bei Verwendung der Vorrichtung 1 die Insekten 8 in dem Behälter 2 zu der Eiablagevorrichtung 4 gelockt, um dort die Insekteneier 7 abzulegen. Die Eiablagevorrichtung 4 ist dabei dementsprechend ausgeführt, um zu verhindern, dass die Insekten 8 vom Behälterinnenraum 3 zum Lockmittel 5 gelangen, und dort die Eier ablegen. Das Lockmittel 5 ist dabei vorzugsweise Wasser und/oder tote Insekten 14. Das Wasser als Lockmittel 5 kann neben flüssig auch als Aerosol vorliegen. Natürlich ist das Lockmittel 5 nicht darauf beschränkt und es können auch unterschiedliche Lockmittel 5 verwendet werden. Weiters kann als das Lockmittel 5 auch ein fermentierter Stoff verwendet werden, genauso wie eine Mischung verschiedener Lockmittel 5. Beispielsweise kann das Wasser als Lockmittel 5 auch mit Zucker oder Essig vermischt werden. Zusätzlich kann die zumindest eine Eiablagevorrichtung 4 auch mit einem weiteren Lockmittel parfümiert sein. Das Lockmittel 5 gibt die Reizstoffe 6 beispielsweise in Form eines Duftstoffes ab, welcher von den Insekten 8 wahrgenommen wird und die Insekten 8 zur Eiablagevorrichtung 4 lockt.

In Fig. 2 ist eine erfindungsgemäße Vorrichtung 1 zur Produktion von Insekteneiern 7 dargestellt. Im Gegensatz zu dem grundlegenden Aufbau der Vorrichtung 1 in Fig. 1, ist die Eiablagevorrichtung 4 in Fig. 2 auf einem Förderband 9 angeordnet, wobei die Eiablagevorrichtung 4 vorzugsweise zumindest teilweise vom Förderband 9 entfernbar ist (z.B. als Segmente). In einer weiteren Ausführungsform kann auch ein umlaufender Gurt des Förderbandes 9 als die Eiablagevorrichtung 4 dienen. Der Behälter 2 weist in Fig. 1 eine Auslassöffnung 10 auf, wobei sich die Eiablagevorrichtung 4 auf dem Förderband 9 durch die Auslassöffnung 10 hindurch erstreckt. Das Lockmittel 5 ist unterhalb dem Förderband 9 angeordnet (nicht sichtbar in Fig. 2), wodurch dieses örtlich von der Eiablagevorrichtung 4 getrennt ist. Weiters sorgt eine Umhausung des Förderbandes 9 dafür, dass das Lockmittel 5 vom Behälterinnenraum 3 abgetrennt ist. Die durch das Lockmittel 5 abgegeben Reizstoffe 6 können, wie mit Fig. 1 beschrieben, durch die Eiablagevorrichtung 4 in den Behälterinnenraum 3 gelangen.

Wie in Fig. 2 dargestellt umfasst der Behälter 2 vorzugsweise eine Insektenfördereinrichtung 11, welche dazu ausgebildet ist, die Insekten 8 in den Behälter 2 zu fördern. Die Insektenfördereinrichtung 11 kann dabei unterschiedlich ausgestaltet sein, z.B. ebenfalls als ein Förderband oder als ein pneumatischer Förderer, welcher die Insekten 8, z.B. mit Unter- oder Überdruck, in den Behälter 2 befördert. Natürlich kann der Behälter 2 auch eine Mehrzahl an Insektenfördereinrichtungen 11 umfassen. In Fig. 2 sind beispielhaft Öffnungen 15 in der Insektenfördereinrichtung 11 vorhanden, durch welche die Insekten 8 in den Behälterinnenraum 3 gelangen können. Die Öffnungen 15 können dauerhaft offen sein, können aber auch z.B. mechanisch, hydraulisch oder pneumatisch geöffnet und geschlossen werden. Die Insekten 8 können auch beispielsweise als Puppen mit der Insektenfördereinrichtung 11 gefördert werden, wobei aus den Puppen, z.B. auf einem Förderband der Insektenfördereinrichtung 11, erwachsenen Insekten 8 (z.B. Fliegen) heranwachsen, welche durch die Öffnungen 15 in den Behälterinnenraum 3 gelangen können. Die Insekten 8 könnten natürlich auch im Behälter 2 heranwachsen.

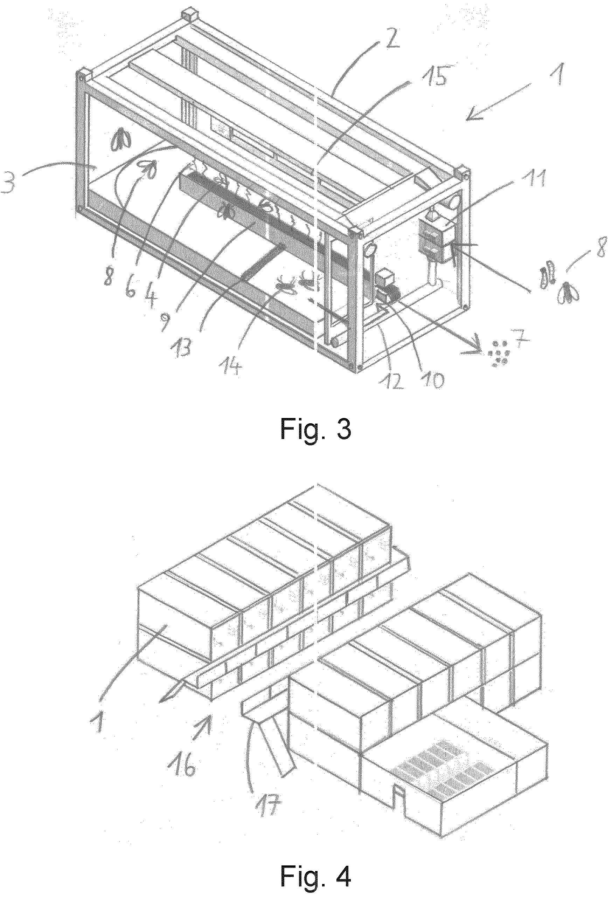

Vorzugsweise weist der Behälter 2 eine Sammeleinrichtung 12 auf, welche dazu ausgebildet ist, tote Insekten 14 (in Fig. 3 dargestellt) in dem Behälter 2 zu sammeln und im Bereich der zumindest einen Eiablagevorrichtung 4 als das zumindest eine Lockmittel 5 anzuordnen. Im Behälter 2 kann dazu beispielsweise eine Abziehvorrichtung 13 (z.B. aus Kunststoff) vorgesehen sein, welche die toten Insekten 14 am Behälterboden abzieht und Richtung Sammeleinrichtung 12 befördert. Die Sammeleinrichtung 12 kann z.B. einen pneumatischen Förderer umfassen, welcher dazu ausgebildet ist, die gesammelten toten Insekten 14 beispielsweise unterhalb dem Förderband 9 als das zumindest eine Lockmittel 5 anzuordnen. Zusätzlich kann die Sammeleinrichtung 12 auch dazu ausgebildet sein, Überreste der Puppen, welche mit der Insektenfördereinrichtung 11 gefördert wurden, zu sammeln, um diese ebenfalls als das zumindest eine Lockmittel 5 anzuordnen.

In Fig. 3 ist ein beispielhaftes Verfahren zur Produktion von Insekteneiern 7 mit der bevorzugten Ausführungsform der Vorrichtung 1 (siehe Fig. 2) dargestellt. Der Behälter 2 wird mit Insekten 8 (z.B. schwarze Soldatenfliegen) gefüllt, wobei diese vorzugsweise über die Insektenfördereinrichtung 11 und z.B. durch die Öffnungen 15 in den Behälterinnenraum 3 gelangen. Die Insekten 8 im Behälterinnenraum 3 werden durch das zumindest eine Lockmittel 5 unterhalb des Förderbandes 9 zur Eiablagevorrichtung 4 gelockt, wobei die Insekten 8 die Insekteneier 7 in die Eiablagevorrichtung 4 ablegen.

Vorzugsweise wird einmal pro Tag die Eiablagevorrichtung 4 auf dem Förderband 9 durch die Auslassöffnung 10 aus dem Behälter 2 gefördert (z.B. einmal die gesamte Länge des Förderbandes 9), um die Insekteneier 7 aus der Eiablagevorrichtung 4 zu entfernen. Dabei können die Insekteneier 7 mechanisch oder pneumatisch oder durch Ausspülen mit einer Flüssigkeit (z.B. mit Wasser) aus der Eiablagevorrichtung 4 entfernt werden. Dazu kann der Behälter 2 eine Vorrichtung, wie etwa ein Luftgebläse, umfassen, welches mit Druckluft die Insekteneier 7 aus der Eiablagevorrichtung 4 entfernt. Eine weitere Möglichkeit wäre es, dass die Eiablagevorrichtung 4 auf dem Förderband 4 kontinuierlich gefördert wird und die Insekteneier 7 dabei aus der Eiablagevorrichtung 4 entfernt werden.

Wie in Fig. 3 dargestellt werden die toten Insekten 14 im Behälter 2 mit einer Sammeleinrichtung 12 gesammelt, wobei eine Abziehvorrichtung 13 die toten Insekten 14 auf dem Behälterboden abzieht und Richtung der Sammeleinrichtung 12 fördert. Die gesammelten toten Insekten 14 werden anschließend als das zumindest eine Lockmittel 5 der Eiablagevorrichtung 4 bereitgestellt, indem die toten Insekten 14 unter dem Förderband 9 angeordnet werden.

In Fig. 4 ist eine bevorzugte Anordnung 16 der erfindungsgemäßen Vorrichtung 1 dargestellt, wobei eine Mehrzahl an Vorrichtungen 1 gestapelt und aneinandergereiht sind. Die Vorrichtung 1 kann dazu Verbindungselemente (nicht dargestellt) aufweisen, welche dazu dienen, Vorrichtungen 1 untereinander zu verbinden. Zusätzlich können Podeste 17 vorgesehen sein, um die Vorrichtungen 1, z.B. für Kontroll- und/oder Wartungsarbeiten, einfach zu erreichen. Die Vorrichtung 1 kann beispielsweise weiters Hebevorrichtungen (z.B. am Behälter 2, nicht dargestellt) umfassen, welche dazu dienen, um die Vorrichtung 1, z.B. mit einem Flurförderzeug oder einem Kran, zu bewegen. Je nach Kundenwunsch und/oder Produktionsvolumen kann eine Mehrzahl an Vorrichtung 1 zur Produktion von Insekteneiern 7 unterschiedlich angeordnet werden.

## Patentansprüche

1. Vorrichtung (1) zur Produktion von Insekteneiern (7), wobei ein Behälter (2) vorgesehen ist, welcher dazu ausgebildet ist, Insekten (8), insbesondere als erwachsene Insekten (8), in einem Behälterinnenraum (3) des Behälters (2) aufzunehmen und die aufgenommenen Insekten (8) daran zu hindern, den Behälter (2) zu verlassen, wobei im Behälter (2) zumindest eine Eiablagevorrichtung (4) vorgesehen ist, welche dazu ausgebildet ist, Insekteneier (7), welche bei Verwendung des Behälters (2) durch die Insekten (8) im Behälter (2) abgelegt werden, aufzunehmen, wobei die zumindest eine Eiablagevorrichtung (4) zumindest teilweise aus dem Behälter (2) entfernbar ist, wobei im Bereich der zumindest einen Eiablagevorrichtung (4) zumindest ein Lockmittel (5) angeordnet ist, welches Reizstoffe (6) für die Insekten (8) abgibt, um bei Verwendung des Behälters (2) die Insekten (8) zum Ablegen der Insekteneier (7) zu der zumindest einen Eiablagevorrichtung (4) zu locken, wobei das zumindest eine Lockmittel (5) von der zumindest einen Eiablagevorrichtung (4) örtlich getrennt ist und vom Behälterinnenraum (3) abgetrennt ist, wobei die vom Lockmittel (5) abgegebenen Reizstoffe (6) durch die zumindest eine Eiablagevorrichtung (4) in den Behälterinnenraum (3) gelangen, **dadurch gekennzeichnet, dass** die zumindest eine Eiablagevorrichtung (4) auf einem Förderband (9) angeordnet ist.

2. Vorrichtung (1) nach Anspruch 1, **dadurch gekennzeichnet, dass** das zumindest eine Lockmittel (5) Wasser und/oder tote Insekten ist.

3. Vorrichtung (1) nach Anspruch 1 oder 2, **dadurch gekennzeichnet, dass** die zumindest eine Eiablagevorrichtung (4) perforiert ist.

4. Vorrichtung (1) nach einem der Ansprüche 1 bis 3, **dadurch gekennzeichnet, dass** die zumindest eine Eiablagevorrichtung (4) zumindest teilweise vom Förderband (9) entfernbar ist.

5. Vorrichtung (1) nach einem der Ansprüche 1 bis 4, **dadurch gekennzeichnet, dass** der Behälter (2) zumindest eine Auslassöffnung (10) aufweist, wobei sich die zumindest eine Eiablagevorrichtung (4) auf dem Förderband (9) zumindest teilweise durch die zumindest eine Auslassöffnung (10) hindurch erstreckt.

6. Vorrichtung (1) nach einem der Ansprüche 1 bis 5, **dadurch gekennzeichnet, dass** als das zumindest eine Lockmittel (5) Wasser unterhalb des Förderbandes (9) angeordnet ist.

7. Vorrichtung (1) nach einem der Ansprüche 1 bis 6, **dadurch gekennzeichnet, dass** der Behälter (2) zumindest eine Insektenfördereinrichtung (11) umfasst, welche dazu ausgebildet ist, die Insekten (8) in den Behälter (2) zu fördern.

8. Vorrichtung (1) nach einem der Ansprüche 1 bis 7, **dadurch gekennzeichnet, dass** der Behälter (2) eine Sammeleinrichtung (12) aufweist, welche dazu ausgebildet ist, tote Insekten (14) in dem Behälter (2) zu sammeln und im Bereich der zumindest einen Eiablagevorrichtung (4) als das zumindest eine Lockmittel (5) anzuordnen.

9. Anordnung (16) einer Mehrzahl an Vorrichtungen (1) nach einem der Ansprüche 1 bis 8, wobei die Mehrzahl an Vorrichtungen (1) aneinandergereiht und/oder gestapelt angeordnet ist.

10. Verfahren zur Produktion von Insekteneiern (7) mit einer Vorrichtung (1) nach einem der Ansprüche 1 bis 8, wobei Insekten (8), insbesondere als erwachsene Insekten, in einem Behälter (2) bereitgestellt werden, **dadurch gekennzeichnet, dass** zumindest ein Lockmittel (5) im Bereich der zumindest einen Eiablagevorrichtung (4) angeordnet wird, **und dass** die Insekten (8) durch das Lockmittel (5) zum Ablegen der Insekteneier (7) zu der zumindest eine Eiablagevorrichtung (4) gelockt werden.

11. Verfahren nach Anspruch 10, **dadurch gekennzeichnet, dass** tote Insekten (14) im Behälter (2) mit einer Sammeleinrichtung (12) gesammelt werden und die toten Insekten (14) als das zumindest eine Lockmittel (5) der zumindest einen Eiablagevorrichtung (4) bereitgestellt werden.

12. Verfahren nach Anspruch 10 oder 11, **dadurch gekennzeichnet, dass** in vorgegebenen Zeitabständen, vorzugsweise einmal pro Tag, die Insekteneier (7) aus der zumindest eine zumindest eine Eiablagevorrichtung (4) entfernt werden.

13. Verfahren nach Anspruch 12, **dadurch gekennzeichnet, dass** die Insekteneier (7) mechanisch oder pneumatisch oder durch Ausspülen mit einer Flüssigkeit aus der zumindest eine Eiablagevorrichtung (4) entfernt werden.

14. Verfahren nach einem der Ansprüche 10 bis 13, **dadurch gekennzeichnet, dass** die Insekten (8) mit zumindest einer Insektenfördereinrichtung (11) in den Behälter (2) gefördert werden.

## Claims

1. An apparatus (1) for producing insect eggs (7), wherein a container (2) is provided that is configured to accommodate insects (8), in particular as adult insects (8), in a container interior (3) of the container (2) and prevents the accommodated insects (8) from escaping from the container (2), wherein at least one egg-laying apparatus (4) is provided in the container (2), which is configured to accommodate insect eggs (7) that are laid in the container (2) by the insects (8) when the container (2) is used, wherein the at least one egg-laying apparatus (4) is at least partially removable from the container (2), wherein in the area of the at least one egg-laying apparatus (4) at least one attractant (5) is arranged that emits stimulants (6) for the insects (8) in order to lure the insects (8) to the at least one egg-laying apparatus (4) to lay the insect eggs (7) when the container (2) is used, wherein the at least one attractant (5) is spatially separated from the at least one egg-laying apparatus (4) and is separated from the container interior (3), wherein the stimulants (6) emitted by the attractant (5) pass through the at least one egg-laying apparatus (4) into the container interior (3), **characterized in that** the at least one egg-laying apparatus (4) is arranged on a conveyor belt (9).

2. The apparatus (1) according to claim 1, **characterized in that** the at least one attractant (5) is water and/or dead insects.

3. The apparatus (1) according to claim 1 or 2, **characterized in that** the at least one egg-laying apparatus (4) is perforated.

4. The apparatus (1) according to any of claims 1 to 3, **characterized in that** the at least one egg-laying apparatus (4) is at least partially removable from the conveyor belt (9).

5. The apparatus (1) according to any of claims 1 to 4, **characterized in that** the container (2) has at least one outlet opening (10), wherein the at least one egg-laying apparatus (4) on the conveyor belt (9) extends at least partially through the at least one outlet opening (10).

6. The apparatus (1) according to any of claims 1 to 5, **characterized in that** water is arranged as the at least one attractant (5) below the conveyor belt (9).

7. The apparatus (1) according to any of claims 1 to 6, **characterized in that** the container (2) comprises at least one insect conveying device (11) that is configured to convey the insects (8) into the container (2).

8. The apparatus (1) according to any of claims 1 to 7, **characterized in that** the container (2) has a collecting device (12) that is designed to collect dead insects (14) in the container (2) and to arrange them in the region of the at least one egg-laying apparatus (4) as the at least one attractant (5).

9. An arrangement (16) of a plurality of apparatuses (1) according to any of claims 1 to 8, wherein the plurality of apparatuses (1) are arranged in a row and/or stacked.

10. A method for producing insect eggs (7) using an apparatus (1) according to any of claims 1 to 8, wherein insects (8), in particular adult insects, are provided in a container (2), **characterized in that** at least one attractant (5) is arranged in the region of the at least one egg-laying apparatus (4), **and in that** the insects (8) are lured to the at least one egg-laying apparatus (4) by the attractant (5) in order to lay the insect eggs (7).

11. The method according to claim 10, **characterized in that** dead insects (14) are collected in the container (2) using a collecting device (12) and the dead insects (14) are provided as the at least one attractant (5) of the at least one egg-laying apparatus (4).

12. The method according to claim 10 or 11, **characterized in that** at predetermined intervals, preferably once a day, the insect eggs (7) are removed from the at least one egg-laying apparatus (4).

13. The method according to claim 12, **characterized in that** the insect eggs (7) are removed from the at least one egg-laying apparatus (4) mechanically or pneumatically or by flushing out with a liquid.

14. The method according to any of claims 10 to 13, **characterized in that** the insects (8) are conveyed into the container (2) by at least one insect conveying device (11).

## Revendications

1. Dispositif (1) pour la production d'œufs d'insectes (7), dans lequel un récipient (2) est prévu, lequel est conçu pour recevoir des insectes (8), en particulier en tant qu'insectes adultes (8), dans un espace intérieur de récipient (3) du récipient (2), et pour empêcher les insectes (8) reçus de quitter le récipient (2), dans lequel au moins un dispositif de dépôt d'œufs (4) est prévu dans le récipient (2), lequel est conçu pour recevoir des œufs d'insectes (7) qui sont déposés dans le récipient (2) lors de l'utilisation du récipient (2) par les insectes (8), dans lequel l'au moins un dispositif de dépôt d'œufs (4) peut être retiré au moins partiellement du récipient (2), dans lequel au moins un appât (5) est disposé dans la zone de l'au moins un dispositif de dépôt d'œufs (4), lequel appât émet des substances attirantes (6) pour les insectes (8) afin d'appâter les insectes (8) vers l'au moins un dispositif de dépôt d'œufs (4) pour le dépôt des œufs d'insectes (7) lors de l'utilisation du récipient (2), dans lequel l'au moins un appât (5) est localement séparé de l'au moins un dispositif de dépôt d'œufs (4) et est séparé de l'espace intérieur de récipient (3), dans lequel les substances attirantes (6) émises par l'appât (5) passent à travers l'au moins un dispositif de dépôt d'œufs (4) dans l'espace intérieur de récipient (3), **caractérisé en ce que** l'au moins un dispositif de dépôt d'œufs (4) est disposé sur une bande transporteuse (9).

2. Dispositif (1) selon la revendication 1, **caractérisé en ce que** l'au moins un appât (5) est de l'eau et/ou des insectes morts.

3. Dispositif (1) selon la revendication 1 ou 2, **caractérisé en ce que** l'au moins un dispositif de dépôt d'œufs (4) est perforé.

4. Dispositif (1) selon l'une des revendications 1 à 3, **caractérisé en ce que** l'au moins un dispositif de dépôt d'œufs (4) peut être au moins partiellement retiré de la bande transporteuse (9).

5. Dispositif (1) selon l'une des revendications 1 à 4, **caractérisé en ce que** le récipient (2) présente au moins une ouverture de sortie (10), dans lequel l'au moins un dispositif de dépôt d'œufs (4) sur la bande transporteuse (9) s'étend au moins partiellement à travers l'au moins une ouverture de sortie (10).

6. Dispositif (1) selon l'une des revendications 1 à 5, **caractérisé en ce que** de l'eau est disposée en dessous de la bande transporteuse (9) en tant que l'au moins un appât (5).

7. Dispositif (1) selon l'une des revendications 1 à 6, **caractérisé en ce que** le récipient (2) comprend au moins un appareil de transport d'insectes (11) qui est conçu pour transporter les insectes (8) dans le récipient (2).

8. Dispositif (1) selon l'une des revendications 1 à 7, **caractérisé en ce que** le récipient (2) présente un appareil de collecte (12) qui est conçu pour collecter des insectes morts (14) dans le récipient (2) et pour les disposer dans la zone de l'au moins un dispositif de dépôt d'œufs (4) en tant que l'au moins un appât (5).

9. Agencement (16) d'une pluralité de dispositifs (1) selon l'une des revendications 1 à 8, dans lequel la pluralité de dispositifs (1) est disposée en ligne et/ou empilée.

10. Procédé pour la production d'œufs d'insectes (7) avec un dispositif (1) selon l'une des revendications 1 à 8, dans lequel des insectes (8), en particulier en tant qu'insectes adultes, sont fournis dans un récipient (2), **caractérisé en ce qu'**au moins un appât (5) est disposé dans la zone de l'au moins un dispositif de dépôt d'œufs (4), et **en ce que** les insectes (8) sont appâtés vers l'au moins un dispositif de dépôt d'œufs (4) par l'appât (5) pour le dépôt des œufs d'insectes (7).

11. Procédé selon la revendication 10, **caractérisé en ce que** des insectes morts (14) sont collectés dans le récipient (2) avec un appareil de collecte (12) et les insectes morts (14) sont fournis en tant que l'au moins un appât (5) de l'au moins un dispositif de dépôt d'œufs (4).

12. Procédé selon la revendication 10 ou 11, **caractérisé en ce qu'**à des intervalles de temps prédéfinis, de préférence une fois par jour, les œufs d'insectes (7) sont retirés de l'au moins un dispositif de dépôt d'œufs (4).

13. Procédé selon la revendication 12, **caractérisé en ce que** les œufs d'insectes (7) sont retirés de l'au moins un dispositif de dépôt d'œufs (4) de manière mécanique ou pneumatique ou par élimination par rinçage avec un liquide.

14. Procédé selon l'une des revendications 10 à 13, **caractérisé en ce que** les insectes (8) sont transportés dans le récipient (2) à l'aide d'au moins un appareil de transport d'insectes (11).
